Europäisches Patentamt

European Patent Office

Office européen des brevets

(19)

(11) Veröffentlichungsnummer: **0 191 945**
**B1**

(12) **EUROPÄISCHE PATENTSCHRIFT**

(45) Veröffentlichungstag der Patentschrift: **23.05.90**

(21) Anmeldenummer: **85116411.1**

(22) Anmeldetag: **21.12.85**

(51) Int. Cl.$^5$: **A 61 B 5/14**

(54) Blutentnahmevorrichtung.

(30) Priorität: **20.02.85 DE 3505782**
**22.11.85 DE 3541335**

(43) Veröffentlichungstag der Anmeldung:
**27.08.86 Patentblatt 86/35**

(45) Bekanntmachung des Hinweises auf die
Patenterteilung:
**23.05.90 Patentblatt 90/21**

(84) Benannte Vertragsstaaten:
**AT BE CH DE FR IT LI NL**

(56) Entgegenhaltungen:
**EP-A-0 031 900**
**GB-A-2 027 168**
**US-A-3 747 812**
**US-A-3 804 113**

(73) Patentinhaber: **KABE Labortechnik Gesellschaft
mit beschränkter Haftung
Jägerhofstrasse 17
D-5223 Nümbrecht-ELsenroth (DE)**

(72) Erfinder: **Bethkenhagen, Jürgen
Heideweg 15
D-5223 Nümbrecht (DE)**
Erfinder: **Kolpe, Dieter
Rosenfeldstrasse 1
D-5276 Wiehl (DE)**
Erfinder: **Bruchmann, Helmut
Am Steegerberg 14
D-5276 Wiehl 2 (DE)**

(74) Vertreter: **Patentanwälte Dipl.-Ing. Alex Stenger
Dipl.-Ing. Wolfram Watzke Dipl.-Ing. Heinz J.
Ring
Kaiser-Friedrich-Ring 70
D-4000 Düsseldorf 11 (DE)**

Courier Press, Leamington Spa, England.

**Beschreibung**

Die Erfindung betrifft und setzt als bekannt voraus eine Blutentnahmevorrichtung mit einer zylindrischen Probenröhre und einem darin verschiebbar angeordneten Kolben sowie mit einer Abschlußkappe, an der ein axial gerichteter Konus exzentrisch angeordnet ist, einem auf dem Konus aufgesetzten Adapter an dem zwei Kanülen befestigt sind, von denen die äußere zum Einführen in eine Vene frei ist und die innere mit einem Schlauchventil verschlossen ist.

Aus der DE—B—18 12 742 ist eine Blutentnahmevorrichtung bekannt, deren Probenröhre evakuiert und mit einem durchstechbaren Stopfen verschlossen ist, der auf das innere Ende der mit dem Schlauchventil verschlossenen Kanüle aufgeschoben wird. Aus der EP—A—031 900 ist eine Blutentnahmevorrichtung entsprechend dem Oberbegriff des Patentanspruches 1 bekannt, deren Abschlußkappe einen axial gerichteten Führungsansatz mit einem durchstechbaren Verschlußstopfen hat.

Aus der US—A—4 341 035 ist eine Blutentnahmevorrichtung bekannt, deren Probenröhre evakuiert und mit einem durchstechbaren Stopfen verschlossen ist, der auf das Ende einer Kanüle aufgeschoben wird. Damit die Blutprobe aus der Probenröhre nicht zurücklaufen kann, ist am inneren Ende ein Ventil angeformt, welches in einer Ausführungsform (Fig. 8 bis 10) aus einem mit getrennt voneinander angeordneten Stegen an der Innenseite des Stopfens angeformten Ventilteller besteht, dessen Außenrand sich am Innenrand einer zylindrischen Öffnung im Stopfen anlegen soll. Zwischen dem Ventilteller und dem von der Kanüle durchstochenen Deckel des Stopfens befindet sich ein verhältnismäßig großer zylindrischer Raum, der auch dann noch mit der Blutprobe teilweise oder ganz gefüllt sein kann, wenn die Probenröhre von der Kanüle abgezogen wird. Die Dichtfläche zwischen dem Ventilteller und dem Stopfen ist sehr groß, so daß ein verhältnismäßig großer Innendruck erforderlich ist, um das Ventil zu schließen.

Alle vorstehend beschriebenen, bekannten Blutentnahmevorrichtungen haben den Nachteil, daß die Probenröhren mit einem Stopfen verschlossen sein müssen, der ohne großen Kraftaufwand von einer Kanüle durchstochen werden kann. Sobald die Probenröhren nach der Blutnahme von der Kanüle abgezogen werden, sind sie nicht mehr dicht, weil ihr Stopfen ein Loch hat. Wenn die Probenröhren abgelegt werden und die Blutprobe unter dem Loch im Stopfen ansteht, ist es nicht zu vermeiden, daß der über die Blutprobe in der Probenröhre wirksamwerdende Dampfdruck die Blutprobe tropfenweise durch das Loch im Stopfen herausdrückt. Dies geschieht auch bei der aus der US-PS 42 49 035 bekannten Blutnahmevorrichtung mit einem auf der Innenseite des durchstechbaren Stopfens angeordneten Ventil.

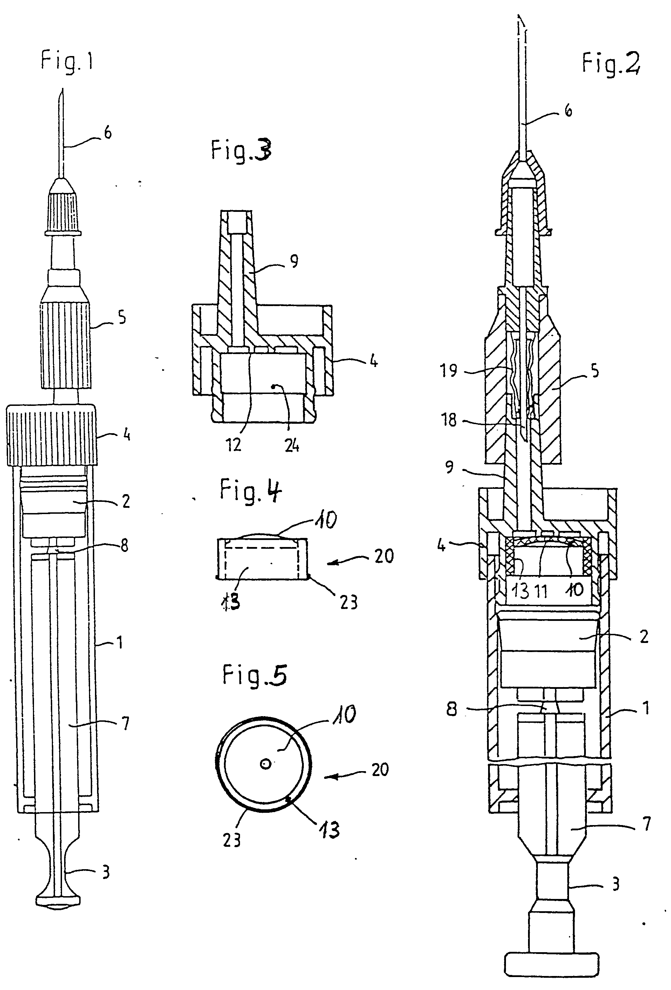

Aus der US—A—3 804 113 ist ein Rückschlagventil bekannt, welches einen Durchfluß nur in einer Richtung auch bei einem sehr kleinen Druckunterschied gestattet. In dem Ausführungsbeispiel gemäß Fig. 3 besteht das Rückschlagventil aus einem einteiligen Gehäuse mit einem exzentrisch angeordneten Einlauf und einem daran anschließenden, zylindrischen Auslauf mit größerem Durchmesser. In den zylindrischen Auslauf ist eine mit einem Ring verbundene Membrane eingesetzt, die in der Mitte eine Bohrung hat und einem konzentrisch am Gehäuse angeordneten Ventilsitz anliegt. Die Membrane ist zwischen ihrem Ring und der Bohrung S-förmig oder balgartig ausgebildet. Eine Membrane mit dieser bekannten Formgestaltung hat die unangenehme Eigenschaft, daß sie beim Abheben vom Ventilsitz unter dem Druck des einströmenden Mediums in Einströmrichtung durchschlägt und danach nur wieder in ihre Schließlage zurückbewegt werden kann, wenn das Medium unter erheblichem Druck wieder zurückströmt. Das kommt aber bei einer Blutentnahmevorrichtung nicht vor.

Schließlich ist aus der GB—A—2 027 168 eine Blutentnahmevorrichtung bekannt, bei der in dem Adapter zwischen den beiden Kanülen in einem verbreiterten Gehäuse ein Rückschlagventil angeordnet ist, welches aus einer kreisscheibenförmigen Membrane mit am äußeren Umfang eingelassenen Ausschnitten und einem im Gehäuse angeordneten, ringförmigen Ventilsitz besteht. Dieses Rückschlagventil im Adapter übernimmt die Aufgabe des Schlauchventiles auf der inneren Kanüle bei einer anderen bekannten Blutentnahmevorrichtung und ist nicht dazu bestimmt und geeignet, die Probenröhre nach dem Abziehen von der inneren Kanüle des Adapters bei geringem Innendruck dicht zu verschließen, um einen Rücklauf oder ein Auslaufen der Blutprobe zu verhindern.

Davon ausgehend liegt der Erfindung die Aufgabe zugrunde, eine einfach konstruierte und handhabbare Blutentnahmevorrichtung zu schaffen, die ebenfalls das Ansetzen mehrerer Probenröhren nacheinander an den gleichen Adapter gestattet, deren Probenröhre aber nach dem Abziehen von der inneren Kanüle des Adapters bei geringem Innendruck dicht verschlossen ist und einen Rücklauf oder ein Auslaufen der Blutprobe verhindert.

Die technische Lösung dieser Aufgabe ist gekennzeichnet durch ein in der Abschlußkappe angeordnetes Rückschlagventil bestehend aus einer elastisch verformbaren Membrane mit einer mittigen Ventilöffnung und einen in die Abschlußkappe eingesetzten, zylindrischen Stützring für die Membrane, wobei die Membrane und der Stützring als einteiliger Membrabring ausgebildet sind und die Membrane in dem Stützring nach innen versetzt angeformt und kugelsegmentförmig so nach außen ausgeformt ist, daß sie den Rand ihres Stützringes etwas überragt, sowie aus einem innen an der Abschlußkappe konzentrisch angeformten, kreisrunden Ventilsitz.

Bei einer wieterentwickelten Ausführungsform ist zur Erleichterung der Montage am Fuß des Stützringes eine außen umlaufende Lippe angeformt und in der Abschlußkappe ein innen umlaufender Absatz eingeformt, auf dem sich die Lippe abstützt und den Stützring mit der Membrane in ihrer montierten Lage fixiert.

Zur Erleichterung der Montage ist am Fuß des Ringes eine außen umlaufende Lippe angeformt und in der Abschlußkappe ein innen umlaufender Absatz eingeformt, auf dem sich die Lippe abstützt und den Ring bzw. die Membrane in ihrer montierten Lage fixiert.

Eine nach dieser technischen Lehre ausgebildete Blutentnahmevorrichtung hat den Vorteil, daß ein Rücklauf oder ein Auslaufen der Blutprobe unter der Wirkung des in der Probenröhre sich einstellenden Dampfdruckes zuverlässig verhindert wird.

Die Erfindung wird anhand der nachfolgenden Beschreibung und der zugehörigen Zeichnung, in welcher eine bevorzugte Ausführungsform einer erfindungsgemäß ausgebildeten Blutentnahmevorrichtung dargestellt worden ist, näher erläutert. In der Zeichnung zeigen:

Fig. 1 eine Blutentnahmevorrichtung in Ansicht;

Fig. 2 dieselbe Blutentnahmevorrichtung im Längsschnitt;

Fig. 3 eine Abschlußkappe ohne Membranring im Querschnitt;

Fig. 4 ein Membranring in Seitenansicht;

Fig. 5 derselbe Membranring in Draufsicht.

Die dargestellte Blutentnahmevorrichtung besteht aus einer zylindrischen Probenröhre 1 mit eine darin verschiebbar angeordneten Kolben 2 mit Kolbenstange 3, einer Abschlußkappe 4, einem Adapter 5 und einer Kanüle 6.

Die Kolbenstange 3 ist mit Stegen 7 in einer viereckigen Aussparung im Boden der Probenröhre 1 geführt. Zwischen dem Kolben 2 und der Kolbenstange 3 ist eine Sollbruchstell 8 vorgesehen, damit die Kolbenstange 3 nach dem Aufziehen einer Blutprobe abgebrochen werden kann.

An die Abschlußkappe 4 ist ein Konus 9 zum Aufsetzen des Adapters 5 angeformt. Der Konus 9 ist mit einer Längsbohrung versehen, die in der Probenröhre 1 mündet. Damit eine aufgezogene Blutprobe nicht durch den Konus 9 ausfließen kann, wenn die Probenröhre 1 abgelegt wird, ist in der Abschlußkappe 4 ein Rückschlagventil angeordnet, welches aus einer elastisch verformbaren Membrane 10 mit einer Ventilöffnung 11 und einem an die Verschlußkappe 4 angeformten, kreisrunden Ventilsitz 12 besteht. Damit die Membrane 10 zuverlässig in der Abschlußkappe 4 festgehalten wird, ist ein Stützring 13 vorgesehen.

In dem Adapter 5 ist eine innere Kanüle 18 angeordnet, auf der ein Schlauchventil 19 befestigt ist, welches mit dem Konus 9 verschoben und dadurch geöffnet werden kann.

Bei der dargestellten Blutentnahmevorrichtung sind die Membrane 10 und der Stützring 13 als ein einteiliger Membranring 20 ausgebildet. Bei diesem Membranring 20 ist die Membrane 10 am äußeren Rand etwas versenkt an einen zylindrischen Stützring 13 angeformt, und zwar mit nach außen gewölbter, Kugelform, so daß die Membrane 10 in der Mitte den Rand des Stützringes leicht überragt. Am Fuß des Stützringes 13 ist eine nach außen zeigende Lippe 23 angeformt.

In die Abschlußkappe 4 ist innen ein Absatz 24 eingeformt, auf dem sich die Lippe 23 des Ringes 22 abstützen kann. Nach dem Einsetzen des Membranringes 20 in die Abschlußkappe 4 liegt die Membrane 10 dem Ventilsitz 12 unter leichter Vorspannung an. Da sich der Membranring 20 mit seiner Lippe 23 auf dem Absatz 24 in der Abschlußkappe 4 abstützt, wird die dichtende Anlage der Membrane 10 am Ventilsitz 12 auch dann aufrechterhalten, wenn die Probenröhre 1 nach dem Abziehen vom Adapter 5 abgelegt wird. Der Membranring 20 bleibt auch in seiner Funktionslage, wenn die Probenröhre 1 zur Weiterverarbeitung der Blutprobe in eine Zentrifuge eingesetzt wird.

Mit einer nach dieser technischen Lehre ausgebildeten Blutentnahmevorrichtung ist es möglich, nach dem Einstechen der Kanüle 6 in eine Vene nacheinander mehrere Probenröhren 1 an den Adapter 5 anzusetzen und mehrere Blutproben für verschiedene Untersuchungen aufzuziehen. Dabei wird das Schlauchventil 19 mit dem Konus 9 der Probenröhre 1 geöffnet. Das Rückschlagventil in der Abschlußkappe 4 öffnet sich selbsttätig, sobald der Kolben 2 mit seiner Kolbenstange 3 verschoben wird. Mit dem in der Probenröhre 1 entstehenden Unterdruck wird die Membrane 10 in eine leicht nach innen gewölbte Lage verformt, so daß die Blutprobe am Ventilsitz 12 vorbei durch die Ventilöffnung 11 in der Membrane 10 und die Öffnung 14 im Stützring 13 in die Probenröhre 1 einströmen kann. Sobald aber der Kolben 2 nicht mehr bewegt oder mal aus Versehen zurückgeschoben werden solte, legt sich die Membrane 10 wieder gegen den Ventilsitz 12 und verschließt damit das Rückschlagventil. Ein Zurückströmen der aufgezogenen Blutprobe ist ausgeschlossen, und zwar sowohl während der Blutentnahme als auch nach dem Abziehen der Probenröhre 1 vom Adapter 5 oder später bei der Weiterverarbeitung der Blutprobe.

Bezugszeichenliste

| | |
|---|---|
| 1 | Probenröhre |
| 2 | Kolben |
| 3 | Kolbenstange |
| 4 | Abschlußkappe |
| 5 | Adapter |
| 6 | Kanüle (äußere) |
| 7 | Steg |
| 8 | Sollbruchstelle |
| 9 | Konus |
| 10 | Membrane |
| 11 | Ventilöffnung |
| 12 | Ventilsitz |
| 13 | Stützring |
| 18 | Kanüle |
| 19 | Schlauchventil |
| 20 | Membranring |
| 23 | Lippe |
| 24 | Absatz |

## Patentansprüche

1. Blutentnahmevorrichtung mit einer zylindrischen Probenröhre (1) und einem darin verschiebbar angeordneten Kolben (2) sowie mit einer Abschlußkappe (4), an der ein axial gerichteter Konus (9) exzentrisch angeordnet ist, einem auf dem Konus aufgesetzten Adapter (5) an dem zwei Kanülen (6, 18) befestigt sind, von denen die äußere zum Einführen in eine Vene frei ist und die innere mit einem Schlauchventil (19) verschlossen ist, gekennzeichnet durch ein in der Abschlußkappe (4) angeordnetes Rückschlagventil bestehend aus einer elastisch verformbaren Membrane (10) mit einer mittigen Ventilöffnung (11) und einem in die Abschlußkappe (4) eingesetzten, zylindrischen Stützring (13) für die Membrane (10), wobei die Membrane (10) und der Stützring (13) als einteiliger Membranring (20) ausgebildet sind und die Membrane (10) in dem Stützring (13) nach innen versetzt angeordnet und kugelsegmentförmig so nach außen ausgeformt ist, daß sie den Rand ihres Stützringes (13) etwas überragt, sowie aus einem innen an der Abschlußkappe (4) konzentrisch angeformten, kreisrunden Ventilsitz (12).

2. Blutentnahmevorrichtung nach Anspruch 1, dadurch gekennzeichnet, daß am Fuß des Stützringes (13) eine außen umlaufende Lippe (23) angeformt ist.

3. Blutentnahmevorrichtung nach den Ansprüchen 1 und 2, dadurch gekennzeichnet, daß in die Abschlußkappe (4) ein innen umlaufender Absatz (24) eingeformt ist, auf dem sich die Lippe (23) abstützt.

## Revendications

1. Dispositif de prélèvement de sang comportant un tube-éprouvette cylindrique (1) dans lequel est logé de manière coulissante un piston (2), un capuchon de fermeture (4) sur lequel est agencé, de façon excentrique, un cône (9) dirigé axialement, et, rapporté sur le cône, un adaptateur (5) sur lequel sont fixées deux canules (6, 18), dont celle extérieure est libre pour être introduite dans une veine, tandis que celle intérieure est obteurée par une valve (19), dispositif caractérisé par un clapet anti-retour agencé dans le capuchon de fermeture (4) et composé d'une membrane (10) déformable élastiquement et comportant une ouverture de soupape centrale (11), d'un anneau cylindrique de maintien (13) destiné à la membrane (10) et agencé dans le capuchon de fermeture (4), la membrane (10) et l'anneau de maintien (13) étant réalisés d'une seule pièce en tant que anneau à membrane (20), et la membrane (10) étant agencée dans l'anneau de maintien (13) en étant décalée vers l'intérieur et dirigée vers l'extérieur en forme de calotte sphérique de manière à dépasser légèrement du niveau du bord de son anneau de maintien (13), ainsi que d'un siège de soupape (12), circulaire, réalisé concentriquement dans le capuchon de fermeture (4).

2. Dispositif de prélèvement de sang selon la revendication 1, caractérisé en ce qu'une lèvre périphérique extérieure (23) est formée au niveau du pied de l'anneau de maintien (13).

3. Dispositif de prélèvement de sang selon les revendications 1 et 2, caractérisé en ce que dans le capuchon de fermeture (4) est formé un épaulement périphérique intérieur (24) sur lequel vient s'appuyer la lèvre (23).

## Claims

1. A blood sampling device comprising a cylindrical sample tube (1) and slidable therein a plunger (2), and also comprising an end cap (4) on which there is eccentrically disposed an axially directed cone (9), an adapter (5) fitted on the cone and having two cannulae (6, 18) secured thereon, the outer cannula being free for introduction into a vein while the inner cannula is closed by a tyre valve (19) characterised by a non-return valve which is disposed in the end cap (4) and which consists of an elastically deformable diaphragm (10) having a central valve aperture (11) and a cylindrical support ring (13) for the diaphragm (10), said ring being inserted into the end cap (4), the diaphragm (10) and the support ring (13) being constructed as a one-piece diaphragm ring (20) and the diaphragm (10) being disposed in the support ring (13) so as to be inwardly offset and being externally formed as a spherical segment such as to project somewhat beyond the edge of its support ring (13) and a circular valve seat (12) integrally formed concentrically on the inside of the end cap (4).

2. A blood sampling device according to Claim 1, characterised in that an external peripheral lip (23) is integrally formed at the base of the support ring (13).

3. A blood sampling device according to Claims 1 and 2, characterised in that an inwardly extending shoulder (24) is formed in the end cap (4), the lip (23) bearing on said shoulder.

Fig.1

Fig.3

Fig.2

Fig.4

Fig.5